(19) Europäisches Patentamt
European Patent Office
Office européen des brevets

(11) Numéro de publication: **0 228 935**
**A1**

# (12) DEMANDE DE BREVET EUROPEEN

(21) Numéro de dépôt: **86402655.4**

(22) Date de dépôt: **28.11.86**

(51) Int. Cl.[4]: **C 07 D 263/04**
C 08 G 18/32, C 07 D 263/06

(30) Priorité: **28.11.85 FR 8517618**

(43) Date de publication de la demande:
**15.07.87 Bulletin 87/29**

(84) Etats contractants désignés:
**AT BE CH DE ES FR GB GR IT LI LU NL SE**

(71) Demandeur: **Société CHRYSO S.A.**
**Route d'Orléans B.P. No. 1**
**F-91380 Chilly Mazarin (FR)**

(72) Inventeur: **Guicquero, Jean-Pierre**
**7 bis rue de la Libération**
**F-94440 Santeny (FR)**

**Le Bourt, Noel**
**12 rue Faraday**
**F-44700 Orvault (FR)**

(74) Mandataire: **Phélip, Bruno et al**
**c/o Cabinet Harlé & Phélip 21, rue de la Rochefoucauld**
**F-75009 Paris (FR)**

Le titre de l'invention a été modifié (Directives relatives à l'examen pratiqué à l'OEB, A-III, 7.3)

## (54) Polyoxazolidines, leur procédé de préparation et leur utilisation comme agents de durcissement, en particulier pour les mastics.

(57) Ces composés, représentés par l'une ou l'autre des formules (Ia) ou (Ib) ci-dessous, sont obtenues par réactions d'aldéhydes ou cétones sur des polybêtaaminoalcools. Ils sont utiles comme agents de durcissement latents dans des compositions à base de polyisocyanates organiques.

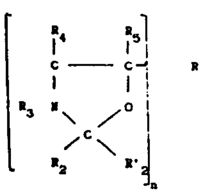 (Ia) 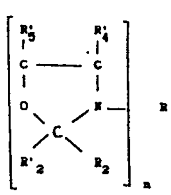 (Ib)

n = 2 à 4; $R_2$, $R'_2$ = notamment H, reste hydrocarboné en $C_1$-$C_{10}$, $R_3$ = reste en $C_1$-$C_{12}$ de valence 1; $R_4$, $R_5$, $R'_4$, $R'_5$ = H ou reste de valance 1; R = reste de fonctionnalité n, et obtenu, pour les composés (Ia), par élimination des n fonctions époxydiques d'un composé polyépoxydique n fonctionnel, et pour les composés (Ib), par élimination des n fonctions $NH_2$ d'une polyamine primaire n fonctionnelle ne comportant ni fonctions uréthane, ni fonctions ester, ni fonctions aldimine, mais comportant obligatoirement au moins une fonction éther dans la chaîne.

EP 0 228 935 A1

## Description

NOUVELLES POLYOXAZOLIDINES-1,3, LEUR PROCEDE DE PREPARATION ET LEUR UTILISATION COMME AGENTS DE DURCISSEMENT, EN PARTICULIER, POUR LES MASTICS.

La présente invention concerne de nouveaux composés à plusieurs cycles oxazolidine-1,3, substitués en positions 3, c'est-à-dire sur l'azote et, éventuellement, sur au moins l'une des positions 2, 4 et 5. L'invention concerne également un procédé pour leur fabrication ainsi que leur utilisation comme agents de durcissement latents pour des systèmes organiques ; notamment pour les mastics à base de polyisocyanates.

Les premières bis-oxazolidines utilisées dans la formulation de mastics polyuréthane étaient notamment celles décrites dans le brevet allemand nº 2 018 233. Elles pouvaient être représentées par la formule générale :

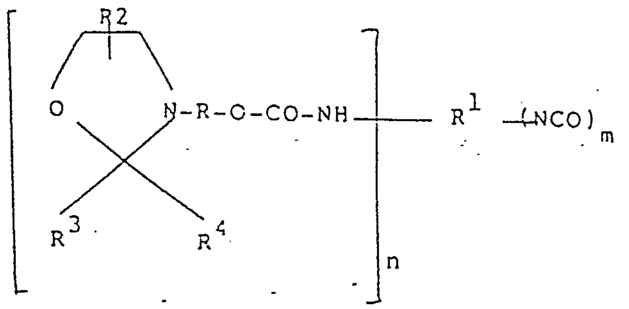

dans laquelle $R^1$ à $R^4$ et R ont diverses significations, R et $R^1$ étant notamment des groupes aliphatiques, et $R^2$ à $R^4$ représentant chacun l'hydrogène ou un reste aliphatique ou aromatique.

Un premier inconvénient de ce type de composés est la présence de groupes ester non modifiés après ouverture du cycle oxazolidine sous l'action de l'humidité atmosphérique et réaction des fonctions libérées sur les fonctions isocyanates des prépolymères des mastics utilisés dans la formulation. Ainsi, les masses d'étanchéité préparées à partir de ces formulations présentaient les désavantages majeurs de toutes les matières synthétiques comportant des groupes esteer, c'est-à-dire une résistance médiocre à l'hydrolyse, surtout en milieu alcalin (cas du contact avec le béton).

Le mode de préparation constituait un deuxième inconvénient ; en effet, la réaction mise en oeuvre (transestérification) possède une cinétique trop lente, ce qui n'est pas avantageux pour une fabrication à l'échelle industrielle.

C'est pourquoi, ont été proposés, dans une demande de brevet français nº 2 286 134 des composés dénommés "uréthanoxazolidines" de formule générale :

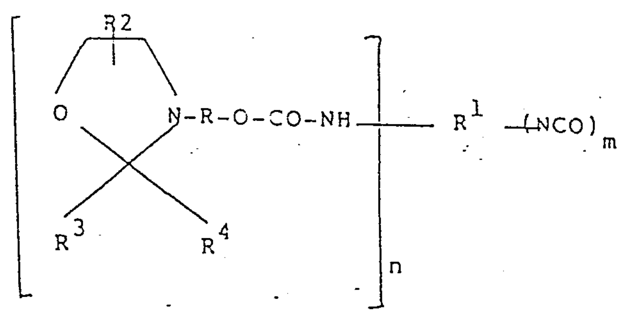

obtenus par réaction de polyisocyanates sur des N-hydroxyalkyloxazolidines synthétisées par condensation de dérivés carbonylés et de bis-(hydroxyalkyl)-amines, comme la diéthanolamine ou la bis-(hydroxy-2 propyl)-amine. Les polyisocyanates utilisés sont bi- ou trifonctionnels, le radical $R^1$ représentant, dans la majeure partie des cas, une chaîne aliphatique. Le composé le plus utilisé de cette série est commercialisé sous la dénomination "DESMODUR 2350" pour lequel :

m=o, n=2, $R=-(CH_2)_2-$, $R^1=-(CH_2)_6-$, $R^2=R^3=H$ et $R^4=$ isopropyle (i-Pr).

On connaît également, par la demande de brevet français nº 2 482 964, des agents de réticulation pour les polyisocyanates, qui sont des composés mixtes oxazolidine-aldimine de formule générale :

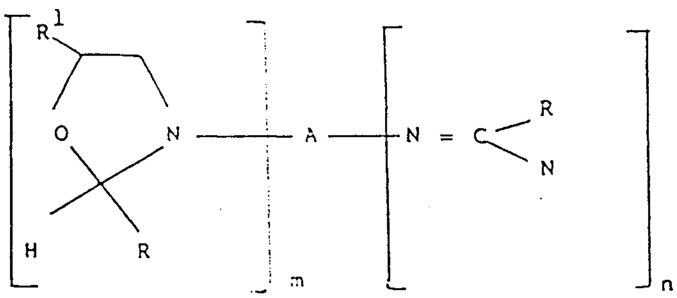

obtenus par réaction d'époxydes sur une polyamine suivie d'une condensation avec un aldéhyde. Par action de l'eau, ces aldimino-oxazolidines libèrent les fonctions hydroxyle, amine secondaire (hydrolyse de l'oxazolidine) et les fonctions amine primaire (hydrolyse de l'aldimine).

La Société déposante a mis au point de nouveaux composés chimiques qui se sont avérés de très bons agents de durcissement des formulations de mastics polyuréthanne, et qui peuvent avantageusement remplacer les agents décrits dans les demandes de brevet sus-indiquées, certains d'entre eux étant remarquables par la grand stabilité de conservation qu'ils confèrent aux formulations de mastics polyuréthanne auxquelles ils sont inclus. On sait en effet qu'il est important que les mélanges préparés à l'avance, dans des cartouches par exemple, et constitués par le prépolymère polyisocyanate et l'agent de durcissement choisi, restent stables jusqu'au moment de la mise en oeuvre.

La présente invention a donc d'abord pour objet des composés chimiques représentés pour l'une ou l'autre des formules générales :

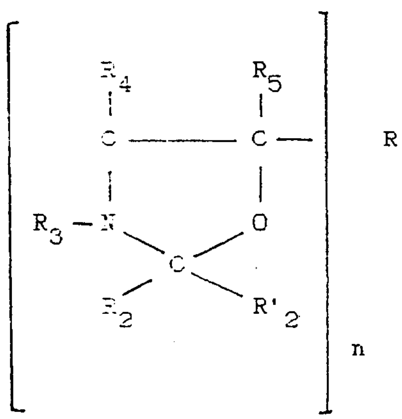

(Ia)

ou

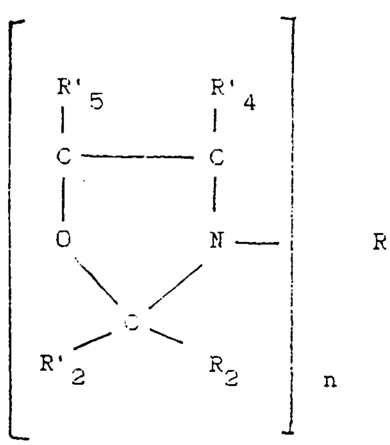

(Ib)

dans lesquelles :

- n est un nombre entier de 2 à 4 ;
- $R_2$ et $R'_2$, identiques ou différents, représentent chacun l'hydrogène ou un reste hydrocarboné en $C_1$ à $C_{10}$ ou encore forment ensemble, avec le carbone en position 2 du cycle oxazolidine, un cycle à 5 ou 6 atomes de carbone ;
- $R_3$ est un reste de valence 1 ne présentant pas d'hydrogène actif selon Zerevinitoff et comportant de 1 à 12

atomes de carbone ;
- $R_4$, $R_5$, $R'_4$, $R'_5$, identiques ou différents, représentent chacun l'hydrogène ou un reste de valence 1, comportant éventuellement un cycle aromatique et ne présentant pas d'hydrogène actif selon Zerevinitoff ; les restes $R_4$ et $R_5$ pouvant former, avec les atomes de carbone en position 4 et 5 du cycle oxazolidine, un cycle hydrocarboné à 5 ou 6 atomes de carbone ; $R_4$ ou $R_5$ pouvant également former, avec un ou plusieurs atomes de R, un cycle alkyle à 5 ou 6 atomes de carbone;
- R est un reste de fonctionnalité n, ne présentant pas d'hydrogène actif selon Zerevinitoff, et obtenu, dans le cas des composés de formule (Ia), par élimination des n fonctions époxydiques :

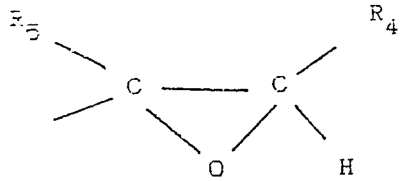

d'un composé polyépoxydique n fonctionnel, et, dans les composés de formule (Ib), par élimination des n fonctions $NH_2$ d'une polyamine primaire n fonctionnelle ne comportant ni fonctions uréthane, ni fonctions ester, ni fonctions aldimine, mais comportant obligatoirement au moins une fonction éther dans la chaîne.

De préférence, dans la formule (Ia) ci-dessus:
- n vaut 2 ou 3
- $R_2$ et $R'_2$, identiques ou différents, représentent chacun l'hydrogène ou un reste hydrocarboné aliphatique en $C_1$ à $C_6$ ;
- $R_3$ comporte de 1 à 7 atomes de carbone ;
- $R_4$, et $R_5$, identiques ou différents, représentent chacun, l'hydrogène ou un reste hydrocarboné aliphatique en $C_1$ à $C_6$ ;
- R représente, un reste bi- ou trivalent, alkylène, arylène, aralkylène, alkylarylène, saturé ou insaturé, pouvant contenir au moins une fonction éther, et pouvant également contenir deux fonctions uréthane ou davantage.

De préférence, dans la formule (Ib) ci-dessus: - n vaut 2 ou 3 ;
- $R_2$ et $R'_2$, identiques ou différents, représentent chacun l'hydrogène ou un reste hydrocarboné aliphatique en $C_1$ à $C_6$ ;
- $R'_4$ et $R'_5$, identiques ou différents, représentent chacun l'hydrogène ou un reste hydrocarboné aliphatique en $C_1$ à $C_6$.

On mentionne également particulièrement les composés répondant à la formule (I'b) :

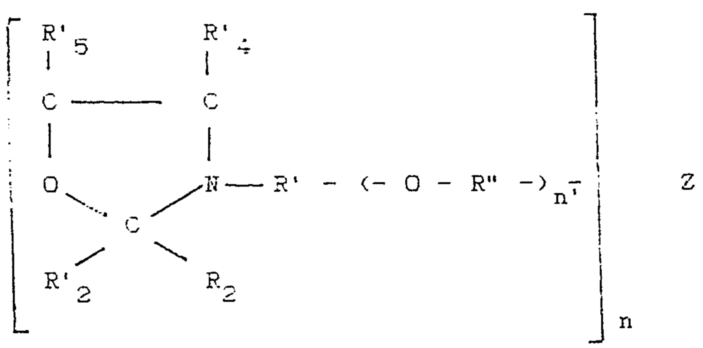

(I'b)

dans laquelle :
- $R_2$, $R'_2$, $R'_4$, $R'_5$ et n ont les mêmes significations que celles indiquées précédemment ;
- R' et R'', identiques ou différents, représentent chacun un reste alkylène inférieur en $C_2$-$C_4$;
- n' est un nombre entier pouvant prendre la valeur 0, les n' pouvant être différents ; et
- Z représente ou bien l'oxygène auquel cas n = 2, ou bien un reste n fonctionnel obtenu par élimination des n hydrogène des n fonctions hydroxyle d'un composé polyhydroxylé n fonctionnel (notamment un polyalcool, un polyphénol, un polyphénolalcool).

On préfèrera encore que, dans tous les cas, n prenne la valeur 2, que $R_2$ soit l'hydrogène et $R'_2$ soit un reste isopropyle.

La présente invention a également ppour objet un procédé de préparation des composés qui viennent d'être définis, ce procédé de préparation étant catactérisé par le fait que l'on fait réagir des bêta-aminoalcools n fonctionnels, n valant de 2 à 4 et dont les fonctions amine sont des fonctions amine secondaire, sur des composés de formule :

$R_2$-CO-$R'_2$ (II)

dans laquelle $R_2$ et $R'_2$ ont les significations indiquées précédemment,
le rapport des fonctions carbonyle aux fonctions bêta-aminoalcools étant compris entre 1 et 10.

De préférence, ce rapport sera compris entre 1 et 1,5.

La réaction de cyclisation, qui est la réaction des bêta-aminoalcools n fonctionnels sur les composés de formule (II) est effectuée, de préférence, en milieu solvant (hexane, cyclohexane, toluène, etc.) et l'eau formée est extraite par entraînement azéotropique.

Lorsque l'on désire préparer des composés de formule (Ia), on prépare les bêta-aminoalcools n fonctionnels en additionnant les monoamines primaires de formule :

$R_3$-$NH_2$ (III)

dans laquelle $R_3$ a la signification indiquée précédemment, sur des composés époxydiques n fonctionnels de formule :

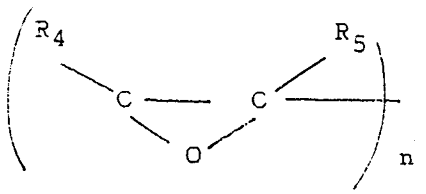

(IV)

dans laquelle, $R_4$, $R_5$, R et n, ont les significations indiquées précédemment,
le rapport des fonctions amine aux fonctions époxy étant supérieur ou égal à 1,1.

Les produits de formule (IV) sont choisis notamment parmi les polyglycidyl-éthers, les polyglycidyl-uréthanes, les polyglycidyl-polyéthers, les polyépoxy-alcanes, les éthers diépoxy-alkyl et les composés mixtes du type glycidyl-éthers d'époxy-alkyl, le motif glycidyl pouvant être substitué (par exemple : méthyl-2 glycidyl-éther).

A titre d'exemples non limitatifs, on peut citer :

polyglycidyl-éthers :

- les diglycidyl-éther des propanes-diols (1,2 et 1,3), de l'éthylèneglycol, des butane-diols (1,2, 1,3, 2,3 et 1,4), du néo-pentyl-glycol, des diphénols, des bisphénols, du bis (hydroxy-méthyl)-2,5 tétrahydrofuranne, des phénols et bisphénols hydrogénés.
- les triglycidyl-éther du glycérol, du triméthylolpropane, du phloroglucinol.

polyglycidyl-uréthanes :

Ce sont les composés d'addition du glycidol ou du méthyl-2 glycidol sur les poly-isocyanates. On peut les écrire :

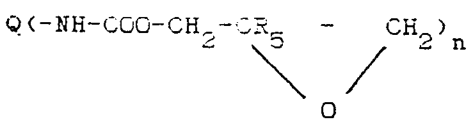

avec $R_5$ = H ou $CH_3$

Le reste Q, de valence n, ne doit pas contenir de fonction biuret. On se limitera ici, aux restes Q de type alkyle, alkyle-aryle, aryle, pouvant contenir des ponts oxygène. Ls polyisocyanates préférés présentent un reste Q à 4 à 18 atomes de carbone. On peut citer :

- le N,N' hexylène-1,6 dicarbamate de glycidyl
- le N,N' toluylène-2,4 dicarbamate de glycidyl
- le N,N' méthylène-3 triméthyl-3,3,5 cyclohexyl-1 dicarbamate de glycidyl.

polyglycidyl-polyéthers

On utilise les glycidyl-éthers des polyols à fonction téléchéliques obtenus par polymérisation ou copolymérisation d'époxydes, tels que oxyde d'éthylène, de propylène, de butylène, de styrène ou d'éther-cyclique, tel que le tétrahydrofuranne, ou obtenus par polyadditions desdits époxydes sur des composés à plusieurs hydrogènes réactifs, tels que les triols, les amines, les polyamines, l'ammoniac.

On cite, à titre d'exemples non limitatifs :

- diglycidyl éther de polyéthylène glycol,
- diglycidyl éther de polypropylène glycol,
- diglycidyl éther de polybutylène glycol-1,4

polyépoxy-alcanes :

- le 1-2,7,8 diépoxy octane
- le 1-2, 8-9 diépoxy p. menthane
- le 2,2 bis(3,4-époxy cyclohexyl)propane

éthers diépoxy-alkyl :
- le diglycidyl-éther
- les isomères du bis(2,3-époxy cyclopentyl)éther

composés mixtes :
- le 6-glycidyl éther du 1,2-époxy hexahydro 4,7-méthanoindane
- les o. et p. époxy cyclopentényl-glycidyl éther
- l'o.glycidyl-phényl glycidyl éther.

On signalera également que l'addition des composés (III) et (IV) peut être effectuée en phase aqueuse ou dans un solvant organique. Par ailleurs, on préfère utiliser un large excès de monoamine, ce qui limite la formation de sous-produits. L'excès d'amine est éliminé par distillation sous pression réduite. Les bêta-aminoalcools n fonctionnels obtenus sont purifiés par toute méthode connue en soi, par exemple, par cristallisation dans un solvant.

Lorsque l'on souhaite préparer un composé de formule (Ib), on peut préparer les bêta-aminoalcools n fonctionnels en additionnant des monoépoxydes de formule :

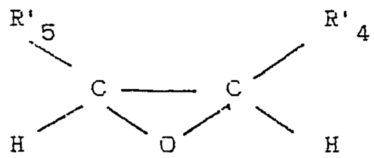 (V)

dans laquelle $R'_4$ et $R'_5$ ont les significations indiquées précédemment,
sur des amines primaires n fonctionnelles de formule :
$(NH_2)_n$-R (VI)
dans laquelle R et n ont les significations indiquées précédemment,
dans un rapport sensiblement stoechiométrique des fonctions époxy aux fonctions amines.

Comme composés de formule (V), on peut utiliser les oxydes d'éthylène, de propylène, de styrène, de cyclohexène, les époxy-(1,2 et 2,3) butanes, l'éther époxy-2,3 propylphénylique, le butyl époxy-2,3 propyléther, etc.

Comme composés de formule (VI), on peut utiliser les éthers amino-2 éthyliques de di- ou tri-ol polyéthers par exemple, le diamino-1,8 dioxa-3,6 octane.

Toujours dans le cas où l'on souhaite préparer des composés de formule (Ib), on peut obtenir les bêta-aminoalcools n fonctionnels en additionnant des monohalohydrines de formule :

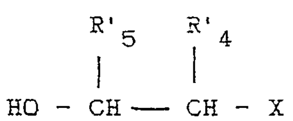 (VII)

dans laquelle $R'_4$ et $R'_5$ ont les significations indiquées précédemment, et X représente un halogène, par exemple le chlore,
sur des amines primaires n fonctionnelles ayant la formule (VI) définie précédemment,
le rapport des fonctions amine aux fonctions halohydrine étant supérieur à 1.

Parmi les composés de formule (VII), on peut mentionner, comme chlorhydrines : le chloro-2 éthanol, le chloro-1 butanol-2, le chloro-1 méthyl-2 propanol-2.

Toujours dans le cas où l'on souhaite préparer des composés de formule (Ib), on peut préparer les bêta-aminoalcools n fonctionnels en additionnant les composés de formule :
$RX_n$ (VIII)
dans laquelle R et n ont les significations indiquées précédemment et X représente un halogène, par exemple le chlore,
sur des mono(bêta-aminoalcools) à fonction amine primaire de formule :

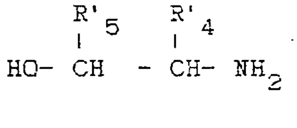 (IX)

dans laquelle $R'_5$ et $R'_4$ ont les significations indiquées précédemment,
la réaction étant conduite avec un excès du composé de formule (IX).

Comme composés de formule (VIII), on peut mentionner, dans le cas où X est le chlore, les éthers bis(chloro-2 éthyliques) de polyols.

Comme composés de formule (IX), on peut mentionner la monoéthanolamine, la monoisopropanolamine,

l'amino-2 butanol-1, l'amino-2 cyclohexanol-1, l'amino-3 butanol-2, l'amino-3 hydroxy-2 propyl phényl éther, le phényl glycinol, le phényl éthanol (ou propanol)amine

La présente invention a également pour objet l'application du composé de formule (Ia) ou (Ib), comme agent de durcissement latent, dans des compositions à base de polyisocyanates organiques. De même, la présente invention porte sur des compositions à base de polyisocyanates organiques, durcissables sous l'action de l'eau ou de l'humidité atmosphérique, caractérisées par le fait qu'elles renferment, à titre d'agent de durcissement, au moins un composé de formule (Ia) ou (Ib), telle que définie ci-dessus.

En règle générale, l'agent de durcissement est mis en oeuvre dans un rapport sensiblement stoechiométrique entre la fonction isocyanate de la composition et les fonctions oxazolidine de l'agent de durcissement.

Les oxazolidines préférées selon l'invention sont celles qui comportent un reste R contenant des fonctions éthers ; en effet, elles procurent une meilleure solubilité et une meilleure dispersion dans le prépolymère à extrémités isocyanate, par suite l'analogie de structure qu'elles présentent avec les polyéther polyols utilisés pour la préparation de ces prépolymères.

Les compositions conformes à la présente invention peuvent, bien entendu, renfermer des produits auxiliaires et additifs usuels.

La Société déposante a pu par ailleurs, mettre en évidence, que la mise en oeuvre de cycles oxazolidine comportant trois ou quatre substituants nécessite l'utilisation d'un catalyseur d'ouverture du cycle oxazolidine (anhydride d'acide organique), dans un rapport de fonctionnalités anhydride d'acide/cycle oxazolidine bien supérieur à celui qui est pratiqué dans le cas où l'on met en oeuvre des cycles oxazolidine bi-substitués.

La présente invention porte donc également sur une composition à base de polyisocyanates organiques, durcissable sous l'action de l'eau ou de l'humidité atmosphérique, renfermant comme agent de durcissement latent, au moins un composé des formules (Ia) et (Ib) indiquées ci-dessus dans lesquelles ($R_4$, $R_5$, $R'_4$ et $R'_5$ sont l'hydrogène, caractérisée par le fait qu'elle renferme en outre, un anhydride d'acide organique, comme catalyseur du cycle oxazolidine, le rapport des fonctionnalités anhydride d'acide/cycle oxazolidine étant sensiblement égal à 1/20.

La présente invention porte aussi sur une composition à base de polyisocyanates organiques, durcissables sous l'action de l'eau ou de l'humidité atmosphérique, renfermant, à titre d'agent de durcissement latent, au moins un composé de formule (Ia) ou (Ib) :

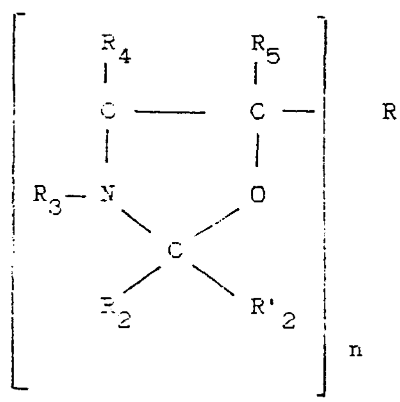

(Ia)

ou

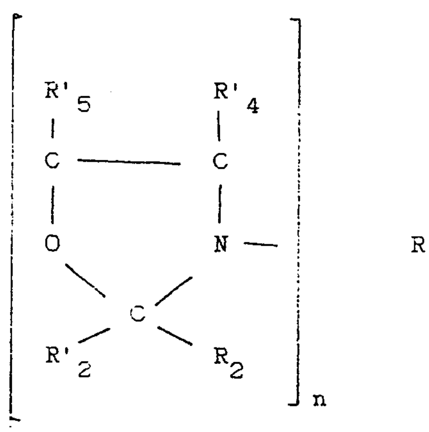

(Ib)

dans lesquelles :

- n est un nombre entier de 2 à 4 ;
- $R_2$, $R'_2$ et $R_3$ ont les significations indiquées précédemment ;
- $R_4$, $R_5$, $R'_4$, $R'_5$ ont les significations indiquées précédemment, au moins l'un parmi $R_4$ et $R_5$ et au moins l'un

parmi $R'_4$ et $R'_5$ étant différent de l'hydrogène ;
- R est un reste de fonctionnalité n, ayant dans le cas des composés de formule (1a) les significations indiquées précédemment, et, dans le cas des composés de formule (Ib) s'obtenant par élimination des n fonctions $NH_2$ d'une polyamine primaire n fonctionnelle.
et, d'autre part, à titre de catalyseur d'ouverture des cycles oxazolidine, au moins un anhydride d'acide organique, caractérisée par un rapport des fonctionnalités anhydride d'acide/cycle oxazolidine sensiblement égal à 1/2.

On a mesuré les constates d'hydrolyse de composés à deux fonctions oxazolidine dans le solvant di(méthoxy-2 éthyl)éther en présence d'acide acétique. Les résultats, rapportés dans le Tableau I ci-après, mettent en évidence que les oxazolidines, ne présentant pas de substituants en positions 4 et 5, peuvent être regroupées : leur constante K de dissociation varient entre $-2{,}13.10^{-3}$ et $-3{,}06.10^{-3}$. Les autres oxazolidines portant au moins un substituant en 3,4, ou 5 ont une constante K de dissociation variant entre $-1{,}33.10^{-4}$ et $-3{,}93.10^{-4}$.

TABLEAU I

| ex: n° | Formule | K ($\Delta^{-1}$) |
|---|---|---|
| 5 | 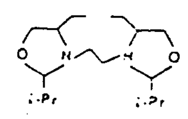 | $-1{,}33\ 10^{-4}$ |
| 1 | 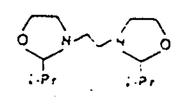 | $-3{,}06\ 10^{-3}$ |
| 4 | 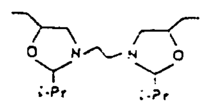 | $-3{,}65\ 10^{-4}$ |
|  | 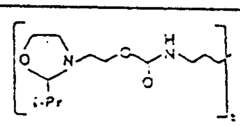 | $-2{,}71\ 10^{-3}$ |
| 2 | 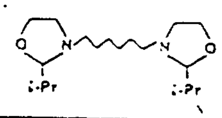 | $-2{,}13\ 10^{-3}$ |
|  | 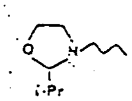 | $-2{,}17\ 10^{-3}$ |
| 7 | 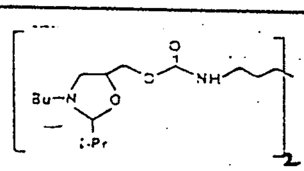 | $-3{,}32\ 10^{-4}$ |
| 6 | 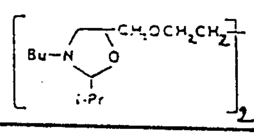 | $-3{,}93\ 10^{-4}$ |
| 3 | 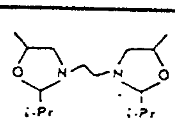 | $-3{,}47\ 10^{-4}$ |

Les exemples qui suivent illustrent l'invention sans toutefois en limiter la portée.

EXEMPLE 1 :

Préparation du bis-(isopropyl-2 oxazolidinyl-3)-1,2 éthane de formule :

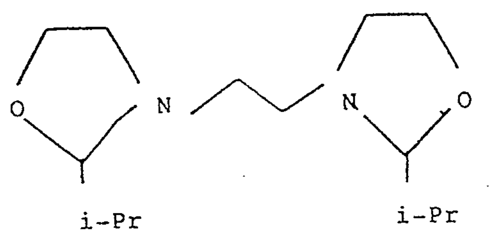

SCHEMA REACTIONNEL:

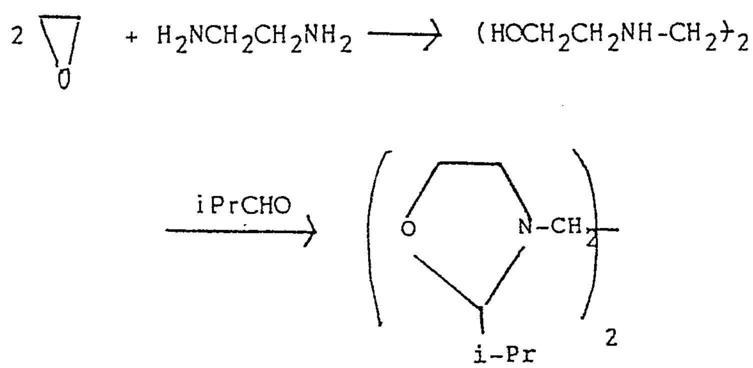


L'éthylène diamine est dissoute dans le méthanol; on y ajoute, à une température inférieure à 15°C, de l'oxyde d'éthylène en quantité stoechiométrique. On chauffe sous agitation jusqu'à disparition de l'ébullition d'époxyde . Le mélange réactionnel est distillé sous pression réduite . Le solvant, la diamine et le composé de monoaddition recueillis en début de distillation pourront être recyclés .

La fraction de coeur, constituée du produit de diaddition (Rendement : 45,9%) distille à 118°C sous 0,01 mbar. Ses caractéristiques sont les suivantes:
RMN$^1$H (D20): 2,65 (t,8H J=5Hz); 3,60(t,4H,J=5Hz); 4,70(s,OH,NH,D20)
RMN$^{13}$C 5CD30D): 47,66 SS; 50,09; 60,35
Analyse: C= 48,59(48,63; H=10,58; (10,88); N=18,68(18,90).

Le bis-(hydroxy-2 éthyl amino)-1,2 éthane obtenu, est dissous dans le toluène à chaud et l'isobutyraldéhyde (distillé),est ajouté (rapport aldéhyde/fonction aminoalcool= 1,25).On porte à reflux, sous courant d'azote, jusqu'à l'obtention de la quantité d'eau calculée, dans le séparateur de Dean-Stark. Le solvant et l'aldéhyde en excès sont éliminés sous vide. Le bis-(isopropyl-2 oxazolidinyl-3)-1,2 éthane peut alors être purifié par distillation. Ses caractéristiques sont les suivantes :
T éb= 67°C sous 0,01 mbar (Rendement : 92%)
RMN $^1$H: 0,85 à 0,95(2d, 12H J=3Hz); 1,60(M,2H); 2,60(m,6H) 3,20(M,2H); 3,80(m,6H).
RMN $^{13}$C: 16,33; 18,82; 31,69; 52,88; 54,22;54,33;64,54; 101,51.

EXEMPLE 2

Préparation du bis(isopropyl-2 oxazolidinyl-3)-1,6 hexane.

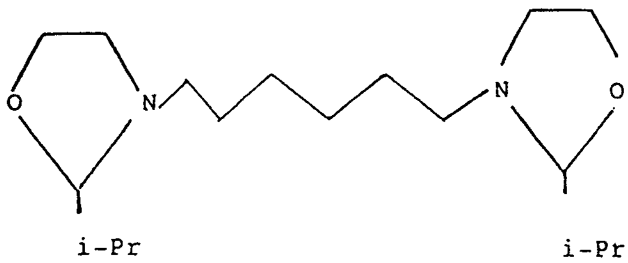

On opère comme dans l'exemple 1

La diamine de départ est le diamino-1,6 hexane dont les caractéristiques sont les suivantes :
T éb.= 135°C sous 0,01 mbar (Rendement = 76%)
RMN 1H: 0,85 à 0,95(2d, 12H,J= 3Hz); 1,40(m,10H); 2,45(m, 8H);3,15(M,2H); 3,75(m4H)
RMN $^{13}$C: 16,34, 18,85; 27,21; 29,34; 31,63; 52,47,54,29; 64,59; 101,38.

EXAMPLE 3:

Préparation du bis(isopropyl-2 méthyl-5 oxazolidinyl-3)-1,2 éthane de formule:

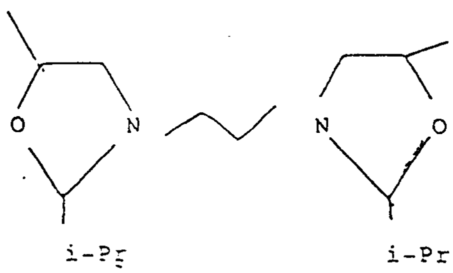

On opère comme dans l'exemple 1. Le monoépoxyde de départ est l'époxy-1,2 propane. Ses caractéristiques sont les suivantes :
T éb. 85°C sous 0,05 mbar (Rendement = 88%)
RMN $^{1}$H: 0,80 à 1,40(m,18H); 1,70(m,2H); 2,20 à 3,10(m,6H); 3,40(m,2H); 3,95(m,4H)
RMN $^{13}$C: 15,78; 15,90; 16,39; 16,54; 18,68; 18,82; 19,15; 19,66; 20,02; 31,50; 31,59; 32,17; 53,77; 54,67; 54,94; 55,09; 59,66; 60,91; 71,01; 71,15 ; 72,71; 100,48; 100,63; 102,14; 102,34.

EXEMPLE 4

Préparation du bis-(isopropyl-2 éthyl-5 oxazolidinyle-3 )-1,2 éthane de formule :

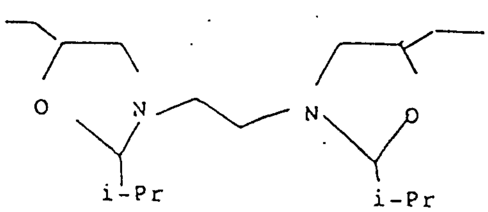

On procède comme dans l'exemple 1 on utilisant l'oxyde de butylène -1,2.

Les caractéristiques du produit obtenu sont les suivantes :
T éb: 82-83°C sous 0,01mbar (Rendement: 85%)
RMN $^{1}$H: 0,90(m,18H); 1,50(m,6H); 2,10 à 3,00(m,7H); 3,35 (m,2H); 3,85(m,3H).
RMN $^{13}$C: 10,14; 15,78; 16,39; 18,82; 27,50; 27,97; 31,45; 31,93; 53,67; 53, 91; 54,76; 57,74; 59,07;76,56; 78,26; 100,42; 101,93.

EXEMPLE 5

Préparation du bis-(isopropyl-2 éthyl-4 oxazolidinyl-3)-1,2 éthane de formule :

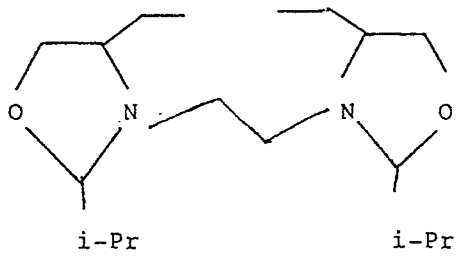

a) Préparation de l'Ethambutol-base

Le chlorhydrate d'Ethanebutol - produit commercialisé par les Laboratoires SOBIO -(100g; 0,36 mole) est mis en suspension dans le chlorure de méthylène (1 litre) , puis refroidi dans un bain de glace + sel.On ajoute, goutte à goutte , une solution de soude (28,85g; 0,72 mole dans 60 ml d'eau), en veillant à ce que la température ne dépasse pas 0°C. A l'issue de l'addition, on maintient l'agitation pendant 1 heure , puis on décante. Après évaporation de la phase organique, on recueille l'Ethambutol-base (Rendement : 73%).

b) Obtention du bis(isopropyl-2 éthyl-4 oxazolidinyl-3)-1,2 éthane

On obtient le produit attendu à partir de l'Ethambutol -base selon le mode opératoire général,en procédant comme indiqué à l'exemple 1.

Le produit attendu possède les caractéristiques suivantes :

T éb: 93-94°C/0,01mbar (Rendement: 89%).

RMN: $^{1}H$: 0,95(m,18H); 1,50(m,6H); 2,60(m,6H); 3,40 à 4,00 (M,6H).

RMN $^{13}C$: 10,62; 16,27; 18,82($CH_3$); 32,72(CH); 27,81; 54,94; 66,30($CH_2$); 69,94(CH); 102,60(OCHN).

EXEMPLE 6

Préparation du bis[(isopropyl-2 n-butyl-3 oxazolidinyl-5) méthoxy]-1,4 butane et du bis[(isopropyl-2 éthyl-3 oxazolidinyl -5 )] méthoxy - 1,4 butane, de formule respective :

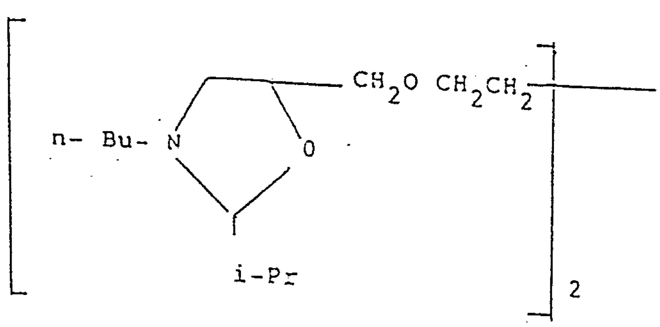

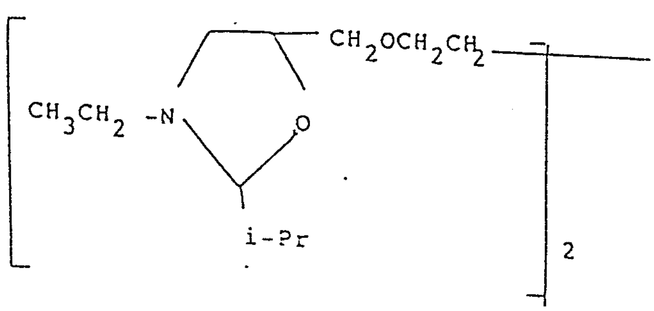

a) Préparation du bis-[(butylamino-3 hydroxy-2 propyl) oxy] -1,4 butane .

La n-butylamine (7,95g); 0,109 moles) est dissoute dans le méthanol (100 ml), puis refroidie dans un bain de glace . On ajoute alors goutte à goutte l'éther diglycidique du butanediol (obtenu à partir d'épichlorhydrine et de butanediol -1,4)(10g; 0,049 mole), on laisse revenir à température ambiante et on porte à reflux pendant 1 heure . Le méthanol et l'excès d'amine sont éliminés , la masse visqueuse résiduelle est laissée à température ambiante jusqu'à cristallisation(quelques jours).Le solide blanc est alors recristallisé dans le toluène. Ses caractéristiques sont les suivantes :

Tf: 62°C(Rendement : 79%)

RMN $^{1}H$: 0,95(t,6H); 1,40(m,8H); 1,70(m,4H); 2,70(m,8H) 2,80(s,8H); 3,80(m,8H); 3,90(M,2H).

RMN $^{13}C$: 14,05(q); 20,51; 26,67; 32,20; 49,71; 52,55; 68,95; 71,65(t); 74,01(d).

b)Préparation du bis-[(éthylamino-3 hydroxy-2 propyl ) oxy] -1,4 butane

La préparation du bis - [(éthylamino-3 hydroxy-2 propyl) oxy] -1,4 butane a été menée selon le même mode opératoire que dans a), en utilisant l'éthylamine en solution aqueuse , mais la cristallisation n'a pu être obtenu . RMN $^{1}H$(produit brut): 1,10(t,6H); 1,65(M,4H); 2,75(m,8H) 3,25(s,4H); 3,50(m,8H); 3,90(M,2H).

c)Préparation des produits finaux

Ces produits sont obtenus à partir des produits préparés aux paragraphes respectivement a) et b) , en procédant comme indiqué à l'exemple 1.
Caractéristiques du bis [(isopropyl-2 n-butyl-3 oxazolidinyl-5)méthoxy] -1,4 butane
Teb: 160° C/0,01 mbar
RMN $^{1}H$: 0,95 (m,18H); 1,55 (m,14H); 2,70(m,8H); 3,50(m,8H); 3,90(M,2H); 4,10(M,2H)
RMN $^{13}C$: 13,94, 16,00, 16,27($CH_3$); 18,90 20,46, 26,46($CH_2$); 31,17, 31,42, 31,51(CH); 53,76 53,92; 55,18, 55,80; ($CH_2$-N) X , 71,35, 71,44,72,82, 73,21, 73,90, 75,51 ($CH_2$-O); 101, 17,101, 97 (OCHN)
Caractéristiques du bis (isopropyl-2 éthyl-3 oxazolidinyl-5) méthoxy -1,4 butane
RMN $^{1}H$ (produit brut): 1,00 (m,18H); 1,55(m,6H);2,70(m,8H) 3,40(m,8H); 3,90(M,2H); 4,10(M,2H).

EXEMPLE 7

Préparation du N,N'-bis-hexylène-1,6 dicarbamate de (isopropyl-2 n-butyl-3 oxazolidinyl-5) méthyle de formule:

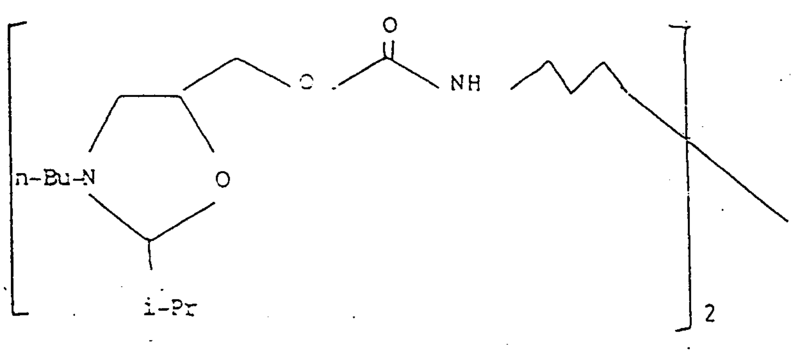

Le glycidol (5g) est dissous dans le toluène (25 ml), puis refroidi avec un bain d'eau froide. On introduit le diisocyanato-1,6 hexane(5g) et le bis-dodécanoate de dibutyl étain (5 mg). Après 15 mn, on observe la cristallisation d'un solide blanc que l'on isole par filtration,puis lavage avec du toluène et séchage sous vide. Le spectre infra-rouge permet de vérifier l'absence dans le produit obtenu de fonctions isocyanate ou alcool n'ayant pas réagi On obtient le N,N' hexylène-1,6 dicarbamate de glycidyl dont on donne le spectre RMN 1H: 1,60(s,8H); 3,00(m,4H); 3,40(d,4H, J=6Hz); 4,05(m,4H); 4,65(m,2H); 5,25(s,2H).

Alors la n-butylamine (5g) est dissoute dans le méthanol (25ml) et refroidie à -5°C. On ajoute goutte à goutte le diépoxy précédent en solution dans un mélange chloroforme-éthanol. On porte à reflux 1 h. L'excès d'amine et les solvants sont alors éliminés à l'évaporateur rotatif. Le diaminoalcool obtenu peut être recristallisé dans le mélange acétone-chlorure de méthylène. On donne son spectre RMN 1H: 0,95(m,6H); 1.30(m,16H); 2,30 à 3,40(m+s,16H); 4,05 (m,6H); 5,20(s,2H).Le produit attendu est préparé suivant le mode opératoire général de préparation des oxazolidines, comme à l'exemple 1, par action de l'isobutyraldéhyde. Toutes traces de solvants et de réactifs sont éliminés par chauffage sous vide (par exemple 100° C, 0,01 mg).

EXEMPLE 8

Application du composé de l'exemple 1 à titre d'agent de durcissement d'une composition à base de polyisocyanates.

On prépare une composition à base de polyisocyanates destinée à servir de masse d'etanchéité du type mastic. Cette composition est formulée comme suit :
-Prépolymère isocyanate 450g
-Alcoylesulfonate de phénol 250g
-Pigment prèdispersé 150g
-Composé de l'exemple 1 40g
-Silice 50g
-Xylène 50g
-Accélérateur 3g

Cette composition est conditionnée dans des cartouches d'aluminium , en vue de son stockage. on a

constaté que pendant tout le temps du stockage (environ 1 an) elle est restée parfaitement stable . Ses caractéristiques sont les suivantes :
-Formation de peau à 20°C à l'air ambiant : 50 mn;
-Allongement à la rupture du système durci : 550% à 2,2 MPa.

EXEMPLE 9

Préparation de l'éther bis (isopropyl-2 oxazolidnyl-3)-2 éthylique) de formule :

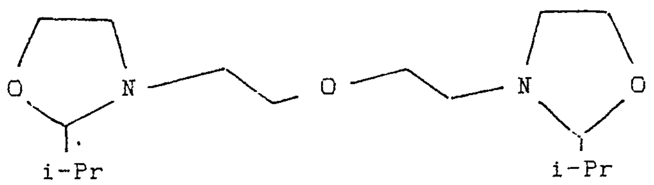

L'éther bis(chloro-2 éthylique) (25,74 g ; 0,18 mole) est dissous dans le tétrahydrofuranne, puis refroidi dans un bain de glace. On ajoute alors progressivement la monoéthanolamine (25,3 g ; 0,41 mole), on laisse revenir à température ambiante sous agitation et l'on porte à reflux pendant une heure. Ensuite, on introduit lentement une solution de soude à 50% (29 g ; 0,36 mole). Le sel formé peut être éliminé par décantation. Le tétrahydrofuranne et l'excès de monoéthanolamine sont éliminés. Le produit attendu est obtenu selon le mode opératoire général, par action de l'isobutyraldéhyde. On obtient un liquide huileux clair, que l'on peut distiller à 135°C sous 0,15 mbar.
Rendement : 82%.

EXEMPLE 10

Préparation de dioxazolidinyl-3 polyoxypropylène de formule générale :

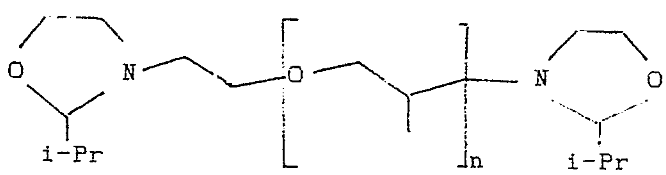

On procède comme à l'exemple 1. La diamine de départ est une polyoxypropylène diamine, à fonctions amine primaire, de formule :
$H_2N-CH_2-CH_2-(-O-CH_2-CH(CH_3)-)_n-NH_2$
par exemple la polypropylènamine ≪Jeffamine D 230 ou D 400≫ de la Société ≪JEFFERSON CHEM.≫.
On obtient des liquides huileux, légèrement colorés en jaune, de viscosité 400 $mm^2/s$ environ à partir de la ≪Jeffamine D 230≫, et de viscosité 730 $mm^2/s$ environ à partir de la ≪Jeffamine D 400≫. Ils présentent, à l'infra-rouge, un faible résidu d'absorption dans la zone 3500-3300 qui ne nuit pas à leur utilisation.

**Revendications**

1 - Composés chimiques représentés par l'une ou l'autre des formules générales:

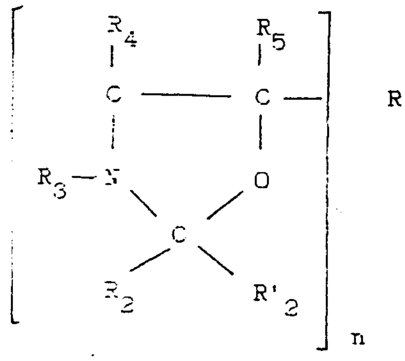

(Ia)

ou

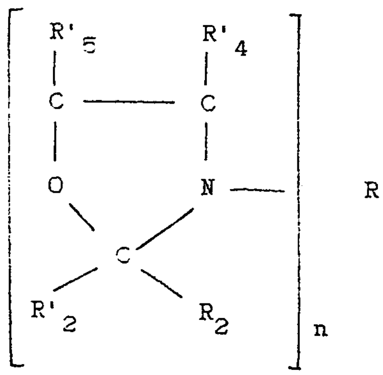

(Ib)

dans lesquelles :
- n est un nombre entier de 2 à 4 ;
- $R_2$ et $R'_2$, identiques ou différents, représentent chacun l'hydrogène ou un reste hydrocarboné en $C_1$ à $C_{10}$ ou encore forment ensemble, avec le carbone en position 2 du cycle oxazolidine, un cycle à 5 ou 6 atomes de carbone ;
- $R_3$ est un reste de valence 1 ne présentant pas d'hydrogène actif selon Zerevinitoff et comportant de 1 à 12 atomes de carbone ;
- $R_4$, $R_5$, $R'_4$, $R'_5$, identiques ou différents, représentent chacun l'hydrogène ou un reste de valence 1, comportant éventuellement un cycle aromatique et ne présentant pas d'hydrogène actif selon Zerevinitoff ; les restes $R_4$ et $R_5$ pouvant former, avec les atomes de carbone en position 4 et 5 du cycle oxazolidine, un cycle hydrocarboné à 5 ou 6 atomes de carbone ; $R_4$ ou $R_5$ pouvant également former, avec un ou plusieurs atomes de R, un cycle alkyle à 5 ou 6 atomes de carbone;
- R est un reste de fonctionnalité n, ne présentant pas d'hydrogène actif selon Zerevinitoff, et obtenu, dans le cas des composés de formule (Ia), par élimination des n fonctions époxydiques :

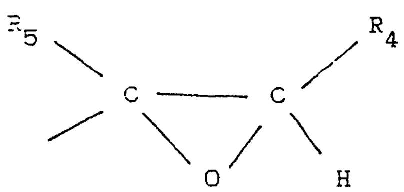

d'un composé polyépoxydique n fonctionnel, et, dans les composés de formule (Ib), par élimination des n fonctions $NH_2$ d'une polyamine primaire n fonctionnelle ne comportant ni fonctions uréthane, ni fonctions ester, ni fonctions aldimine, mais comportant obligatoirement au moins une fonction éther dans la chaîne.

2 - Composés selon la revendication 1, caractérisés par le fait que, dans la formule (Ia),
- n vaut 2 ou 3;
- $R_2$ et $R'_2$, identiques ou différents, représentent chacun l'hydrogène ou un reste hydrocarboné aliphatique en $C_1$ à $C_6$ ;
- $R_3$ comporte de 1 à 7 atomes de carbone ;
- $R_4$ et $R_5$, identiques ou différents, représentent chacun l'hydrogène ou un reste hydrocarboné aliphatique en $C_1$ à $C_6$ ;
- R représente un reste bi- ou trivalent, alkylène, arylène, aralkylène, alkylarylène, saturé ou insaturé, pouvant contenir au moins une fonction éther, et pouvant également contenir deux fonctions uréthane ou

davantage.

3 - Composés selon la revendication 1, caractérisé par le fait que, dans la formule (Ib),
- n vaut 2 ou 3 ;
- $R_2$ et $R'_2$, identiques ou différents, représentent chacun l'hydrogène ou un reste hydrocarboné aliphatique en $C_1$ à $C_6$ ;
- $R'_4$ et $R'_5$, identiques ou différents, représentent chacun l'hydrogène ou un reste hydrocarboné aliphatique en $C_1$ à $C_6$.

4 - Composés selon l'une des revendications 1 et 3, caractérisé par le fait qu'ils répondent à la formule (I'b) :

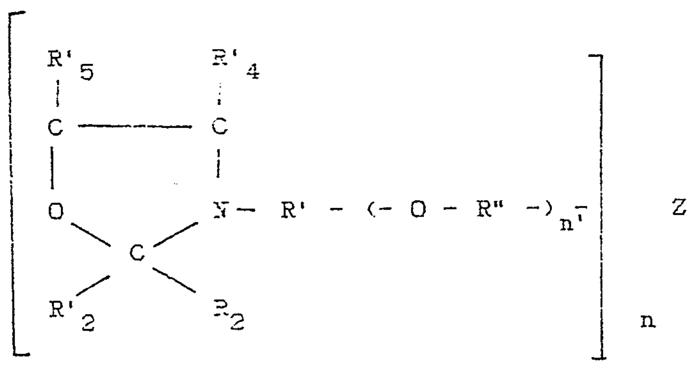

(I'b)

dans laquelle :
- $R_2$, $R'_2$, $R'_4$, $R_5$ et n ont les mêmes significations que celles indiquées à la revendication 1 ;
- R' et R'', identiques ou différents, représentent chacun un reste alkylène inférieur en $C_2$-$C_4$;
- n' est un nombre entier pouvant prendre la valeur 0, les n' pouvant être différents ; et
- Z représente ou bien l'oxygène auquel cas n = 2, ou bien un reste n fonctionnel obtenu par élimination des n hydrogènes des n fonctions hydroxyle d'un composé polyhydroxylé n fonctionnel.

5 - Composés selon l'une des revendications 1 à 4, caractérisés par le fait que n vaut 2, $R_2$ est l'hydrogène et $R'_2$ est un reste isopropyle.

6 - Procédé de préparation d'un composé tel que défini à l'une des revendications 1 à 5, caractérisé par le fait que l'on fait réagir des bêta-aminoalcools n fonctionnels, n valant de 2 à 4, et dont les fonctions amine sont des fonctions amine secondaire, sur des composés de formule :

$R_2$-CO-$R'_2$ (II)

dans laquelle $R_2$ et $R'_2$ ont les significations indiquées à la revendication 1,
le rapport des fonctions carbonyle aux fonctions bêta-aminoalcool étant compris entre 1 et 10.

7 - Procédé selon la revendication 6, caractérisé par le fait que le rapport des fonctions carbonyle aux fonctions bêta-aminoalcool est compris entre 1 et 1,5.

8 - Procédé selon l'une des revendications 6 et 7, destiné à la préparation des composés de formule (Ia), caractérisé par le fait que les bêta-aminoalcools n fonctionnels sont obtenus par addition de monoamines primaires de formule :

$R_3$-$NH_2$ (III)

dans laquelle $R_3$ a la signification indiquée à la revendication 1,
sur des composés époxydiques n fonctionnels de formule :

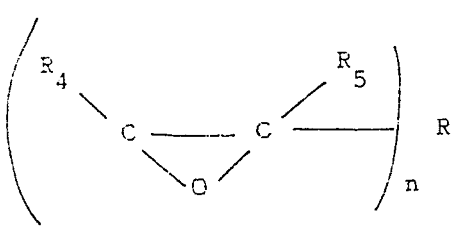

(IV)

dans laquelle $R_4$, $R_5$, R et n ont les significations indiquées à la revendication 1,
le rapport des fonctions amine aux fonctions époxy étant supérieur ou égal à 1,1.

9 - Procédé selon l'une des revendications 6 et 7, destiné à la préparation des composés de formule (Ib), caractérisé par le fait que les bêta-aminoalcools n fonctionnels sont obtenus par addition de monoépoxydes de formule :

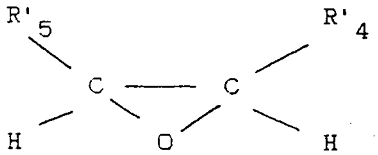

(V)

dans laquelle R'4 et R'5 ont les significations indiquées à la revendication 1, sur des polyamines primaires n fonctionnelles de formule :

$(NH_2)_n$-R (VI)

dans laquelle R et n ont les significations indiquées à la revenidcation 1,

dans un rapport sensiblement stoechiométrique des fonctions époxy aux fonctions amine.

10 - Procédé selon l'une des revendications 6 et 7, destiné à la préparation du composé de formule (Ib), caractérisé par le fait que les bêta-aminoalcools n fonctionnels sont obtenus par addition de monohalohydrines de formule :

HO-CH($R'_5$)-CH($R'_4$)-X (VII)

dans laquelle $R'_4$ et $R'_5$ ont les significations indiquées à la revendication 1, et X représente un halogène, par exemple le chlore,

sur des polyamines primaires n fonctionnelles ayant la formule (VI) définie à la revendication 9, le rapport des fonctions amine aux fonctions halohydrine étant supérieur à 1.

11 - Procédé selon l'une des revendications 6 et 7, destiné à la préparation des composés de formule (Ib), caractérisé par le fait que les bêta-aminoalcools n fonctionnels sont obtenus par addition des composés de formule :

$RX_n$ (VIII)

dans laquelle R et n ont les significations indiquées à la revendication 1, et X représente un halogène, par exemple le chlore,

sur des mono-bêta-aminoalcools à fonction amine primaire de formule :

HO-CH($R'_5$)-CH($R'_4$)-$NH_2$ (IX)

dans laquelle $R'_4$ et $R'_5$ ont les significations indiquées à la revendication 1, la réaction étant conduite avec un excès du composé de formule (IX).

12 - Procédé selon la revendication 8, caractérisé par le fait que l'on choisit les composés de formule (IV) parmi les polyglycidyl-éthers, les polyglycidyl-uréthanes, les polyglycidyl-polyéthers, les polyépoxy-alcanes, les éthers diépoxy-alkyl, et les composés mixtes du type glycidyl-éthers d'époxy-alkyl, le motif glycidyl pouvant être substitué.

13 - Procédé selon la revendication 9, caractérisé par le fait que l'on choisit les composés de formule (VI) parmi les éthers amino-2 éthyliques de di- ou tri-ol-polyéthers.

14 - Application des composés tels que définis à l'une des revendications 1 à 5, à titre d'agents de durcissement latent dans des compositions à base de polyisocyanates organiques.

15 - Composition à base de polyisocyanates organiques, durcissable sous l'action de l'eau ou de l'humidité atmosphérique, caractérisée par le fait qu'elle renferme, à titre d'agent de durcissement, au moins un composé de formule (Ia) ou (Ib), tel que défini à l'une des revendications 1 à 5.

16 - Composition selon la revendication15, renfermant, comme agent de durcissement latent, au moins un composé de formule (Ia) ou (Ib), dans laquelle $R_4$, $R_5$, $R'_4$, $R'_5$ sont l'hydrogène, caractérisée par le fait qu'elle renferme, en outre, comme catalyseur d'ouverture des cycles oxazolidine, au moins un anhydride d'acide organique, le rapport des fonctionnalités anhydride d'acide/cycle oxazolidine étant sensiblement égal à 1/20.

17 - Composition à base de polyisocyanates organiques, durcissable sous l'action de l'eau ou de l'humidité atmosphérique, renfermant, à titre d'agent de durcissement latent, au moins un composé de formule (Ia) ou (Ib) :

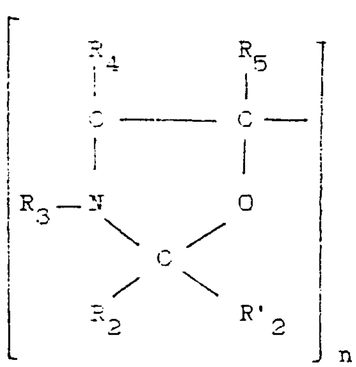

(Ia)

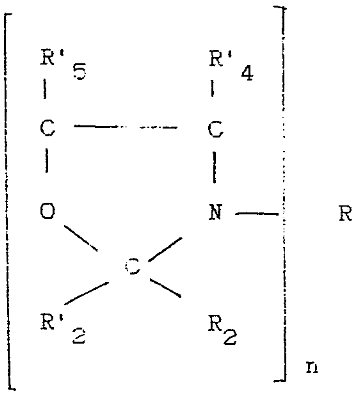

(Ib)

dans lesquelles :
- n est un nombre entier de 2 à 4 ;
- $R_2$, $R'_2$ et $R_3$ ont les significations indiquées à la revendication 1 ;
- $R_4$, $R_5$, $R'_4$, $R'_5$ ont les significations indiquées à la revendication 1, au moins l'un parmi $R_4$ et $R_5$ et au moins l'un parmi $R'_4$ et $R'_5$ étant différent de l'hydrogène ;
- R est un reste de fonctionnalité n, ayant dans le cas des composés de formule (Ia) les significations indiquées à la revendication 1, et, dans le cas des composés de formule (Ib), s'obtenant par élimination des n fonctions $NH_2$ d'une polyamine primaire n fonctionnelle,
et, d'autre part, à titre de catalyseur d'ouverture des cycles oxazolidine, au moins un anhydride d'acide organique, caractérisée par un rapport des fonctionnalités anhydride d'acide/cycle oxazolidine sensiblement égal à 1/2.

Office européen des brevets

# RAPPORT DE RECHERCHE EUROPEENNE

Numero de la demande

EP 86 40 2655

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | CHEMICAL ABSTRACTS, vol. 74, no. 15, 12 avril 1971, page 406, résumé no. 75962b, Columbus, Ohio, US; K.D. PETROV et al.: "Synthesis of 1,2-bis(beta-hydroxyethylamino)ethane and its derivatives", & IZV. VYSSH. UCHEB. ZAVED., KHIM. KHIM. TEKHNOL. 1970, 13(11), 1622-4<br>* Résumé *<br>--- | 1,3,4 | C 07 D 263/04<br>C 08 G 18/32<br>C 07 D 263/06 |
| D,Y | US-A-4 002 601 (M. HAJEK)<br>* En entier *<br>--- | 1,14-17 | |
| D,Y | FR-A-2 482 964 (SIKA AG)<br>* Revendications *<br>--- | 1,14-17 | **DOMAINES TECHNIQUES RECHERCHES (Int. Cl.4)** |
| Y | US-A-4 002 637 (SHELDON N. LEWIS)<br>* Revendications *<br>--- | 1,14-17 | C 07 D 263/00<br>C 08 G 18/00 |
| Y | US-A-3 864 335 (WILLIAM D. EMMONS)<br>* Revendications *<br>--- | 1,14-17 | |
| D,Y | FR-A-2 077 401 (ROHM & HAAS)<br>* Revendications *<br>----- | 1,14-17 | |

Le présent rapport de recherche a été établi pour toutes les revendications

| Lieu de la recherche | Date d'achèvement de la recherche | Examinateur |
|---|---|---|
| LA HAYE | 25-02-1987 | HENRY J.C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant